# FASCICULE DE BREVET EUROPEEN

(11) **EP 3 709 962 B1**
(45) Date de publication et mention de la délivrance du brevet: **26.01.2022**
(21) Numéro de dépôt: 18803350.0
(22) Date de dépôt: 05.11.2018
(51) Int. Cl.: A61K 8/49, A61Q 3/02

(54) **COMPOSITION ANHYDRE COSMETIQUEMENT ACCEPTABLE POUR VERNIS A ONGLES**
WASSERFREIE KOSMETISCHE ZUSAMMENSETZUNG ZUR VERWENDUNG ALS NAGELLACK
ANHYDROUS COMPOSITION COSMETICALLY ACCEPTABLE FOR USE AS NAIL POLISH

(30) Priorité: 13.11.2017 FR 1760656
(43) Date de publication de la demande: 23.09.2020
(73) Titulaire: International Lacquers, 3225 Bettembourg (LU)
(72) Inventeur: DEROSIER, Frédéric, 57680 Corny Sur Moselle (FR); CISNEROS, Robin, 57070 Saint-Julien-Les-Metz (FR); EDDOUMY, Fatima, 57840 Ottange (FR); DEME, Alexandre, 57710 Tressange (FR); DYLEWICZ, Carole, 57180 Terville (FR)
(74) Mandataire: Cabinet Bleger-Rhein-Poupon
(86) Numéro de dépôt international: PCT/EP2018/080196
(87) Numéro de publication internationale: WO 2019/091923

(56) Documents cités:
- EP-A1- 0 024 114
- EP-A1- 0 931 538
- EP-A1- 2 923 735
- DATABASE GNPD [Online] MINTEL; octobre 2017 (2017-10), "Nail Lacquer", XP002780764, Database accession no. 5126697

## Description

### Domaine technique

La présente invention entre dans le domaine des compositions cosmétiques.

L'invention concerne particulièrement une composition cosmétique anhydre, applicable sur les ongles ou les faux ongles, qui soit non dangereuse pour la santé des utilisateurs et pour l'environnement.

### Etat de la technique

Les vernis à ongles sont généralement utilisés pour maquiller les ongles ainsi que les faux ongles. Les vernis à ongles sont habituellement composés d'un agent filmogène, d'un solvant, et éventuellement d'un ou plusieurs plastifiants, d'une ou plusieurs résines, d'un ou plusieurs agents de rhéologie, des agents thixotropants, et d'un ou plusieurs additifs notamment de type colorants, nacres, agents anti UV.

Pour répondre aux exigences des utilisateurs, les vernis à ongles doivent satisfaire plusieurs critères :
- une bonne application : lorsque le vernis à ongles est appliqué sur l'ongle, il doit former une surface lisse, sans stries, avec une épaisseur constante afin d'obtenir un film et une coloration homogènes,
- un étalement facilité sur toute la surface de l'ongle pour rendre son utilisation pratique,
- une brillance accrue et qui persiste dans le temps,
- des temps de séchage et de durcissement sur l'ongle très courts,
- une bonne tenue du vernis à ongles sur l'ongle : il est en effet souhaité que le vernis s'écaille le moins possible afin de conserver un aspect esthétique.

De nouvelles formulations sont sans cesse développées pour essayer d'améliorer l'une ou l'autre de ces propriétés.

En plus des exigences susmentionnées, les produits cosmétiques se doivent d'être sans danger pour l'environnement et la santé des utilisateurs.

En ce sens, l'industrie du cosmétique est régie par le règlement CE N°1223/2009 du Parlement Européen et du Conseil du 30 novembre 2009 relatif aux produits cosmétiques.

La tendance étant d'obtenir un produit cosmétique de qualité, répondant à la fois aux exigences des utilisateurs susmentionnés et respectant ledit règlement. Il convient d'avoir des produits cosmétiques qui soient les moins toxiques possibles à la fois pour l'environnement et les utilisateurs.

Ainsi, le marché des produits cosmétiques de type vernis se doit de respecter le souhait des utilisateurs d'avoir des produits de qualité, sans risque pour leur santé et pour l'environnement et contenant peu ou pas de substances nocives ou prohibées.

Sur ce point, il a été constaté dans le cadre de l'utilisation normale d'un produit cosmétique de type vernis à ongles, la présence, à l'état de traces, de nitrosamines ou composés nitrosés.

Les nitrosamines sont des produits de réaction, se formant majoritairement de façon non intentionnelle.

Les nitrosamines sont issues de la réaction de nitrosation entre un précurseur de type substrat nitrosable et un agent nitrosant en condition acide.

On entend par « substrat nitrosable » des substrats tels que les amines primaires, amines secondaires, ou les sels d'ammonium quaternaires, et par « agent nitrosant » » des substrats tels que des nitrites.

Dans les compositions de vernis à ongles connues, de la nitrosamine peut se former et se retrouver à l'état de traces.

En effet, les « substrats nitrosables » peuvent se former dans le vernis suite à son vieillissement ou encore être issus de ses composés habituels, tel que l'organo-modifiant présent dans les argiles.

De la même manière, les « agents nitrosants » apparaissent suite au vieillissement du vernis, notamment suite à la dégradation de la nitrocellulose qui est un composé fréquemment utilisé pour apporter de la brillance et un film dur au vernis.

Ainsi, il existe un risque de formation de nitrosamines dans les vernis à ongles connus.

Selon le règlement REACH, certaines nitrosamines peuvent être considérées, selon leur forme et leur teneur dans le produit, comme des substances cancérigènes de catégorie 1B.

En particulier, les nitrosamines telles que les diméthylnitrosamines (NDMA), 1-methyl-3-nitro-1-nitrosoguanidine (MNNG), 2,2-(nitrosoimino)bisethanol (NDELA) ou encore la nitrosodipropylamine (NDPA) sont considérées comme des substances cancérigènes toxiques.

Dans le domaine des cosmétiques et selon le règlement CE N°1223/2009, la teneur en nitrosamines ne doit pas dépasser les 0,05 mg/kg dans les diverses matières premières présentant une fonction amine et la teneur maximale en amine secondaire doit être inférieure à 0,5%.

Ainsi, dans le domaine des cosmétiques des vernis à ongles et selon le règlement CE N° 1223/2009, il est nécessaire de réaliser une évaluation de la sécurité des traces de substances prohibées de type nitrosamines par un toxicologue afin de pouvoir en particulier obtenir des autorisations de mise sur le marché du vernis à ongles et que ladite composition réponde au règlement CE N° 1223/2009.

Par conséquent, afin de pouvoir répondre au règlement CE N°1223/2009, il existe un réel besoin de mieux maitriser la présence de nitrosamines dans les compositions cosmétiques de vernis à ongles. En d'autres termes, pendant la conservation de ces vernis, il convient d'éviter, ou au moins de contrôler, la formation possible des nitrosamines au cours du temps, ceci sans modifier directement ou indirectement les propriétés intrinsèques du vernis. Il convient donc de trouver une solution pour éviter ou limiter la formation de nitrosamines au cours du temps dans une composition cosmétique de vernis sans affecter tous les autres critères requis par l'utilisateur tels que la brillance, la couvrance, la tenue sur l'ongle, des temps de séchage et durcissement courts, un étalement et une application rapides et faciles. Ainsi, il est nécessaire que la solution soit inerte vis-à-vis des composants habituels du vernis et sans effet sur ses propriétés intrinsèques exigées par les utilisateurs.

### Objet de l'invention

En conséquence, l'objet de la présente invention est de fournir une composition anhydre cosmétique pour vernis à ongles limitant ou évitant la formation de nitrosamines au cours du temps, tout en maintenant ses autres propriétés, à savoir une bonne brillance, couvrance, tenue sur l'ongle, ainsi que des temps de séchage et durcissement courts, un étalement et une application rapides et faciles. Plus particulièrement, il convient de trouver une composition anhydre de vernis à ongles, c'est-à-dire un vernis à ongles répondant aux critères et propriétés susmentionnés.

### Description générale de l'invention

La présente invention a pour but de pallier les inconvénients de l'état de la technique et de résoudre le problème mentionné ci-dessus, en proposant une composition anhydre cosmétiquement acceptable pour vernis à ongles comprenant au moins un solvant organique, au moins un agent filmogène, au moins un plastifiant, au moins un agent thixotrope, caractérisée en ce qu'elle comporte en outre au moins une substance thixotrope inhibitrice de la nitrosamine.

On entend par le terme « cosmétiquement acceptable » une composition cosmétique compatible pour l'application sur le corps humain, en particulier sur les ongles ou la peau et qui est sans danger pour la santé physique et physiologique de l'utilisateur.

On entend par le terme « substance thixotrope inhibitrice de la nitrosamine » une substance chimique qui détruit la nitrosamine déjà présente et qui bloque également sa formation dans la composition, et dont la propriété physique d'écoulement, qualifiée par son « indice de thixotropique » varie en fonction du temps.

L'indice thixotropique est une indication sur la rhéologie du milieu et sa capacité à reconstituer le réseau permettant de garantir la stabilité des particules insolubles dans le temps. Il est généralement admis qu'un « indice thixotropique » élevé est le garant d'une plus grande stabilité dans le temps au niveau du comportement anti-sédimentant et, en même temps, d'une bonne fluidité lors de l'application. L'indice thixotropique est calculé en mesurant le rapport entre la viscosité à 60 rpm dit « V2 » et la viscosité à 6 rpm dit « V3 ». Ce rapport V2/V3 étant équivalent à l'utilisation de la composition par un utilisateur qui secoue la composition de l'invention avant son utilisation « V2 » et qui la laisse au repos « V3 ».

Selon l'invention, l'indice thixotropique de la substance inhibitrice de la nitrosamine inclus dans ladite composition est compris au moins entre 1,2 et entre 4,5.

Ainsi, ladite substance thixotrope inhibitrice de la nitrosamine permet de limiter ou d'empêcher la formation de nitrosamines au cours du temps dans ladite composition cosmétique de type vernis. En conséquence, tout risque pour la santé de l'utilisateur et pour l'environnement dû à la présence de la nitrosamine est limité. En outre, ladite composition va alors répondre au règlement CE N°1223/2009 et être valablement mise sur le marché.

De plus, selon une autre caractéristique de l'invention, ladite substance thixotrope inhibitrice de la nitrosamine consiste en une ou plusieurs molécule(s) choisie(s) parmi les molécule(s) appartenant à au moins l'une des familles suivantes
- la famille des hydroxypyran-4-ones de formule générique (I) : 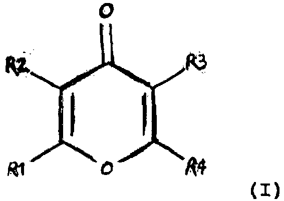 dans laquelle :
- au moins un des radicaux R1, R2, R3 ou R4 consiste en un groupement -OH, ou en un groupement -OR' avec R' étant un éther ou un ester,
- le ou les autres radicaux restants consistant en un groupement -OH, -OR', -H, un alkyl, une amine, un groupement aromatique ou un groupement aliphatique avec ou sans hétéroatome ; et
- la famille des hydroxyfuran-3-one, de formule générique (II) : 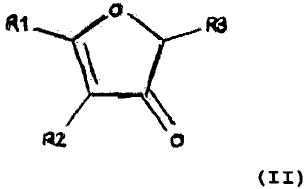
dans laquelle :
- au moins un des groupements R1, R2 ou R3 consiste en un groupement -OH, ou en un groupement -OR' étant un éther ou un ester,
- le ou les autres groupements restants consistant en un groupement -OH, -OR', -H, un alkyl, une amine, un groupement aromatique ou un groupement aliphatique avec ou sans hétéroatome.

La famille des 4-hydroxymethyldioxolanones, de formule générique (III) : 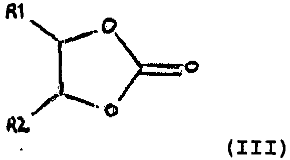 dans laquelle, les radicaux R1 et R2 sont identiques ou différents, R1 et/ou R2 consistant en un groupement -H, un alkyl, une amine, un éther, un ester, un amide, un groupement aromatique ou un groupement aliphatique avec ou sans hétéroatome, est aussi décrite dans la présente demande. Les substances couvertes par la formule (III) sont considérées comme des substances thixotropes inhibitrice de la nitrosamine, mais sont exclues de la présente invention telle que revendiquée.

Par exemple, les molécules de la famille des hydroxypyran-4-ones de formule générique (I) peuvent être celles listées dans le tableau 1 ci-dessous :

**Tableau 1**

| | |
|---|---|
| 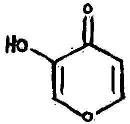 | 3-hydroxypyran-4-one |
| 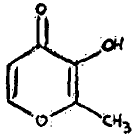 | 3-hydroxy-2-methylpyran-4-one ou maltol |
| 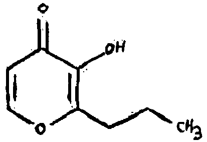 | 3-hydroxy-2-propylpyran-4-one |
| 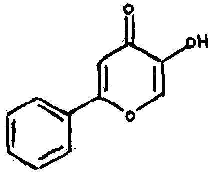 | 5-hydroxy-2-phenylpyran-4-one |
| 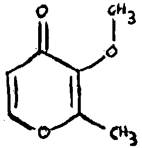 | 3-methoxy-2-methylpyran-4-one |
| 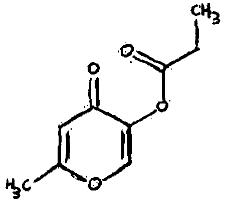 | Propanoate de 6-methyl-4-oxopyran-3-yl |

Selon un mode de réalisation avantageux, les molécules de la famille des hydroxypyran-4-ones de formule générique (I) peuvent présenter des radicaux R1, R2, R3 ou R4 tous identiques, ces radicaux étant alors soit un groupement _OH soit un groupement _OR', tel que susmentionné.

Selon un autre mode de réalisation, les molécules de la famille des hydroxypyran-4-ones de formule générique (I) peuvent présenter des radicaux R1, R2, R3 ou R4 tous différents, cependant au moins l'un d'entre eux sera un radical possédant soit un groupement _OH soit un groupement _OR' , tel que susmentionné.

Par exemple, les molécules de la famille des hydroxyfuran-3-one, de formule générique (II), peuvent être celles listées dans le tableau 2 ci-dessous :

**Tableau 2 :**

| | |
|---|---|
| 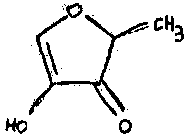 | 4-hydroxy-2-methylfuran-3-one |
| 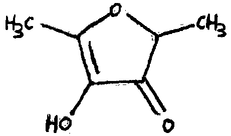 | 4-hydroxy-2,5-dimethylfuran-3-one ou furaneol |
| 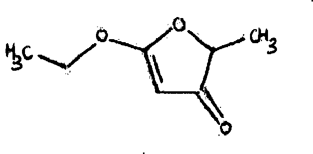 | 5-ethoxy-2-methylfuran-3-one |
| 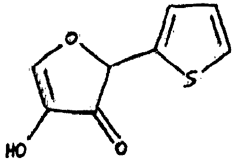 | 4-hydroxy-2-(thiophen-2-yl)furan-3-one |
| 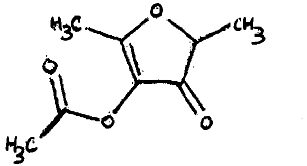 | Ethanoate de 2,5-dimethyl-3-oxopyran-4-yl |

Selon un mode de réalisation avantageux, les molécules de la famille des hydroxyfuran-3-ones, de formule générique (II) peuvent présenter des radicaux R1, R2 ou R3 tous identiques, ces radicaux étant alors soit un groupement OH, soit un groupement _OR', tel que susmentionné.

Selon un autre mode de réalisation, les molécules de la famille des hydroxyfuran-3-ones, de formule générique (II) peuvent présenter des radicaux R1, R2 ou R3 tous différents, cependant au moins l'un d'entre eux sera un radical possédant soit un groupement _OH, soit un groupement OR', tel que susmentionné.

Les molécules de la famille des 4-hydroxymethyldioxolanones, de formule générique (III) peuvent par exemple être choisies dans la liste du tableau 3 ci-dessous :

**Tableau 3 :**

| | |
|---|---|
| 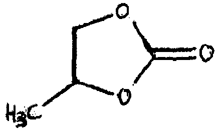 | Carbonate de propylène |
| 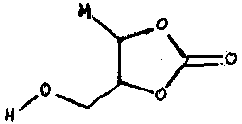 | 4-hydroxymethy-1,3-dioxolan-2-one |
| 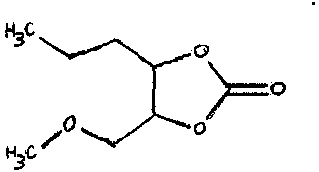 | 4-methoxymothy-5-butyl-1,3-dioxolan-2-one |
| 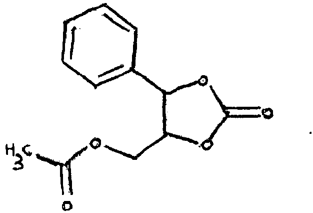 | (2-oxo-5-phenyl-1,3-dioxolan-4-yl)methyl |

Selon un mode préférentiel de réalisation de l'invention, ladite substance thixotrope inhibitrice de la nitrosamine consiste en du maltol et/ou du furaneol.

En effet, ces deux composés ont été sélectionnés spécifiquement dans les familles de molécules susmentionnées pour leurs compétences spécifiques et ciblées à l'encontre de la fabrication de la nitrosamine ou de son élimination et pour leur inertie vis-à-vis des autres constituants de la composition anhydre de l'invention.

Plus spécifiquement, chacune des deux molécules susmentionnées prises individuellement a pour effet d'empêcher la réaction de nitrosation, en agissant notamment sur les précurseurs de type substrats nitrosables susmentionnés et les agents nitrosants.

De plus, chacune de ces deux molécules permet également de détruire la nitrosamine déjà formée au sein de la composition. Ainsi, l'effet inhibiteur est à la fois curatif et préventif.

En outre, il a été constaté une synergie de l'effet inhibiteur de la nitrosamine du maltol, et du furaneol c'est pourquoi chacun de ces composés a été spécifiquement sélectionné comme inhibiteur de la nitrosamine.

Avantageusement encore, chacun de ces deux composés est inerte vis-à-vis de l'activité des autres constituants de la composition anhydre de l'invention, sauf pour l'effet thixotrope.

En effet, de manière avantageuse, il a été démontré que le maltol, et le furaneol ont, en plus de leur propriété d'inhibition de la nitrosamine, un effet thixotrope. La combinaison de ces deux molécules permet au sein de la composition de l'invention d'augmenter davantage l'indice thixotropique donc de favoriser encore la stabilité de la composition dans le temps.

Selon un mode de réalisation particulier de l'invention, ladite substance thixotrope inhibitrice de la nitrosamine représente entre 0,00001 % et 25 % en poids par rapport au poids total de ladite composition anhydre cosmétiquement acceptable pour vernis à ongles. Ainsi, pour que ladite composition entre dans la réglementation et ne soit pas toxique pour l'utilisateur et l'environnement, seule une proportion de substance thixotrope minime, représentant moins de ¼ de la composition totale, est nécessaire.

Selon une autre caractéristique de l'invention, ladite composition anhydre comporte au moins un solvant organique.

Ledit au moins un solvant organique, est choisi parmi le toluène, le xylène, l'acétate d'éthyle, l'acétate de butyle, l'acétate d'isobutyle, l'acétate de propyle, l'acétate de méthyle, une cétone, un ester, l'isopropanol, le butanol le diacétone alcool, l'éthanol dénaturé avec la méthyléthylcétone ou le diéthyléther, les hydrocarbures linéaires ou cycliques, de préférence le cyclohexane, l'heptane ou n'importe quelle combinaison de ceux-ci.

Le solvant organique pourra être choisi en fonction de ses propriétés physico-chimiques et en considération des autres composants présents dans la composition anhydre pour vernis à ongles. Quoi qu'il en soit, il sera inerte vis-à-vis de l'action inhibitrice et thixotrope de la substance thixotrope inhibitrice de la nitrosamine.

En outre, de façon préférée, ledit au moins un solvant organique représente entre 10% et 90% en poids par rapport au poids total de ladite composition anhydre cosmétiquement acceptable pour vernis à ongles.

Cette quantité étant variable en considération des autres composants de la composition anhydre de l'invention.

De préférence le solvant organique est l'acétate d'éthyle, l'acétate de butyle ou une combinaison de ceux-ci.

Selon une autre caractéristique de l'invention, ladite composition anhydre comporte au moins un agent filmogène.

Selon l'invention, ledit agent filmogène se limite aux agents filmogènes utilisables dans des compositions anhydres cosmétiquement acceptables pour vernis à ongles, c'est-à-dire à des agents filmogènes acceptables et connus de l'homme du métier pour fabriquer des vernis à ongles.

Ledit au moins un agent filmogène est choisi parmi la cellulose, un dérivé de cellulose, une résine vinylique, une résine polyester, une résine epoxy-tosylamide, une résine acrylique, un styrène acrylique, une résine de copolymère ou n'importe quelle combinaison de ceux-ci.

Un agent filmogène seul ou une combinaison d'agents filmogènes peut être utilisé(e) dans la composition anhydre pour vernis à ongles.

Avantageusement, ledit au moins un agent filmogène représente entre 5 et 40 % en poids par rapport au poids total de ladite composition anhydre cosmétiquement acceptable pour vernis à ongles.

De préférence, l'agent filmogène est la nitrocellulose.

Selon un mode de réalisation particulier, les résines pouvant être utilisées en tant qu'agents filmogènes sont les suivantes :
- Les résines tosylamides/formaldéhydes ou résines toluènesulfonamides/formaldéhydes,
- Les résines tosylamides/époxy ou résines toluènesulfonamides/époxy,
- Copolymères acides adipiques/néopentyls glycols/anhydridse trimellitiques,
- Copolymères d'anhydrides phtaliques/glycérines/glycidyls décanoates,
- Copolymères d'anhydrides phtaliques/anhydrides trimellitiques/glycols,
- Copolymères de glycérine/acides phtaliques,
- Polymères et copolymères d'acrylates,
- Copolymères d'acrylates et de styrènes,
- Copolymères styrènes/acrylates/acrylonitriles,
- Sucroses acétates isobutyrates,
- Résines polyvinylbutyraux.

De préférence, les compositions ne comprennent pas ou très peu (< 2 %) de composés à base de copolymères d'acrylates, de copolymères d'acrylates et de styrènes ou de copolymères styrènes/acrylates/acrylonitriles.

En plus de leur action collante, la plupart de ces résines additionnelles ont également une action plastifiante. Les résines additionnelles peuvent être utilisées dans la formule, seules ou sous la forme de mélanges, dans des quantités allant de 3 à 20% en poids sec, de préférence 5 à 12% en poids sec, en particulier de 7 à 10% en poids sec par rapport au poids total de la composition.

Selon une autre caractéristique de l'invention, ladite composition anhydre comporte au moins un plastifiant.

Selon l'invention, ledit plastifiant se limite aux plastifiants utilisables dans des compositions anhydres cosmétiquement acceptables pour vernis à ongles, c'est-à-dire à des plastifiants couramment utilisés par l'homme du métier pour fabriquer des vernis à ongles.

Ledit au moins un plastifiant est choisi parmi l'acétyl tributyl citrate, l'acétyl triéthyl citrate, le tributyl citrate, le triéthylcitrate, le dibutyl phtalate, le triphénylphosphate, la triacétine, le triméthyl pentanyl diisobutyrate, la triéthylhexanoïne, le sucrose benzoate, le dibutyl adipate, le diéthyl phtalate, le diisobutyl adipate, le diisopropyl adipate, le dipropylène glycol dibenzoate, le N-éthyl toluène sulfonamide, ses isomères ortho et para, le N-(2-hydroxypropyl) benzène sulfonamide et le N-(n-butyl) benzène sulfonamide ou n'importe quelle combinaison de ceux-ci.

Les plastifiants sont des composés ayant une température d'ébullition élevée qui ne s'évaporent pas lors du séchage de la composition de l'invention de type vernis à ongles. Ils sont utilisés pour moduler la dureté du film formé afin d'obtenir les caractéristiques physico-chimiques du film souhaitées.

Ledit plastifiant représente entre 1 et 20% en poids par rapport au poids total de ladite composition anhydre cosmétiquement acceptable pour vernis à ongles.

Selon une autre caractéristique de l'invention, ladite composition anhydre comporte au moins un agent thixotrope.

Selon l'invention, ledit agent thixotrope se limite aux agents thixotropes utilisables dans des compositions anhydres cosmétiquement acceptables pour vernis à ongles, c'est-à-dire à des agents thixotropes connus de l'homme du métier dans le domaine de la fabrication des vernis à ongles.

Ledit au moins un agent thixotrope choisi parmi les silices pyrogénées et/ou silices hydrophiles et/ou hydrophobes et/ou argiles type bentone, hectorite, kaolin, avec ou sans organo-modifiant(s) ou n'importe quelle combinaison de ceux-ci.

De préférence, ledit au moins un agent thixotrope représente entre 0,05 et 10% en poids par rapport au poids total de ladite composition anhydre cosmétiquement acceptable pour vernis à ongles.

Ces agents thixotropants ont pour effet d'améliorer la fluidité de la composition pour vernis à ongles en augmentant au maximum son indice thixotropique.

L'agent thixotrope diffère de la substance thixotrope inhibitrice de la nitrosamine en ce qu'il ne présente pas une action inhibitrice de la nitrosamine, mais uniquement une action thixotrope au sein de la composition anhydre cosmétique pour vernis à ongles de l'invention.

Au contraire, la substance thixotrope inhibitrice de la nitrosamine présente une double action à la fois « thixotrope » et « d'inhibition de la nitrosamine ».

Plus spécifiquement, la substance thixotrope inhibitrice de la nitrosamine, notamment celle appartenant à la famille des hydroxypyran-4-ones de formule générique (I) et à la famille des hydroxyfuran-3-ones, de formule générique (II) susmentionnée, permet d'activer le ou les agent(s) thixotrope(s) .

En d'autres termes, la substance thixotrope inhibitrice de la nitrosamine, par exemple le maltol, ou le furaneol et n'importe laquelle des combinaisons possibles de ces composés, active l'agent thixotrope en contribuant à son exfoliation, lui donnant ainsi des propriétés thixotropes accrues.

Ainsi, en plus d'avoir un effet de limiter ou éviter la formation de nitrosamine au cours du temps au sein de ladite composition de vernis à ongles de l'invention, ladite substance thixotrope inhibitrice de la nitrosamine agit en synergie avec l'agent thixotrope pour favoriser et augmenter son action thixotrope. Ainsi, ladite substance thixotrope inhibitrice de la nitrosamine est également un activateur du système thixotropant.

Selon une autre caractéristique de l'invention, ladite composition anhydre peut avantageusement comporter également un ou plusieurs additifs choisis parmi les agents rhéologiques, les absorbeurs UV, les modificateurs de surface et les agents dits « traitants » et les agents de coloration.

Par exemple, pour colorer ou fournir des effets particuliers, la composition anhydre pour vernis à ongles peut comprendre un ou plusieurs agents de coloration choisi(s) parmi les pigments, les nacres, les glitters (paillettes) et/ou les particules métalliques. Ces agents de coloration fournissent l'aspect esthétique recherché par les utilisateurs de vernis à ongles. Ces agents de coloration peuvent produire des effets très différents tels que « irisé », « métallisé », « nacré », « miroir », « crème », « pailleté », « craquelé », « ma », « néon », « fluorescent », « holographique », etc...

En particulier, les pigments utilisés peuvent être sélectionnés parmi le groupe constitué par le dioxyde de titane (CI 77891), l'oxyde de fer noir (CI 77499), l'oxyde de fer rouge (CI 77491 ), le DC Red 6 Ba Lake (CI 15850), le DC Red 7 Ca Lake (CI 15850:1), le DC Red 34 Ca Lake (CI 15880), le FDC Yellow 5 Al Lake (CI 19140), le FDC Blue 1 Al Lake (CI 42090), le bleu ultramarine (CI 77007), le DC Violet 2 (CI 60725), le DC Black 2 (CI 77266), etc.

Les nacres sont également utilisables dans l'invention. Les nacres sont des particules minérales de différentes natures recouvertes d'une ou plusieurs couches d'oxydes (oxydes de titane ou oxydes de fer) ou encore de pigments. Les nacres peuvent se présenter sous la forme d'une poudre ou de plaque de tailles variées. Parmi les nacres utilisables dans l'invention, on peut citer :
- les nacres préparées à partir de mica naturel et ayant subi des opérations de coating successives,
- les nacres préparées à partir de mica synthétique appelé également « synthetic fluorphlogopite »,
- les nacres préparées à partir de calcium sodium borosilicate,
- les nacres préparées à partir d'oxyde de silice.

Les glitters (paillettes) utilisables dans l'invention peuvent être préparés à partir de plusieurs types de matériaux comme les films polyesters comportant en surface une couche métallique, le polyéthylène téréphtalate, le polybutylène téréphtalate, les copolymères d'acrylates, les copolymères d'acrylates ou encore l'aluminium.

Selon un mode de réalisation de l'invention, la composition anhydre pour vernis à ongles comprend une quantité d'agents de coloration comprise de 0,1 % à 20 % en poids sec et de préférence de 0,5 % à 12 14 % en poids sec par rapport au poids total de la composition.

De plus, ladite composition de l'invention peut également comporter des absorbeurs UV en tant qu'additifs.

Parmi les absorbants UV, on citera les absorbants de lumière UV qui permettent de protéger la formulation ou les agents colorants utilisés dans l'invention des rayonnements UV. Parmi les protecteurs UV utilisables dans l'invention, on citera : la benzophénone-1 (CAS 131-56-6), la benzophénone-3 (CAS 131-57-7), le benzyl salicylate (CAS 118-58-1), l'étocrylène (CAS 5232-99-5), le drométrizole (CAS 2440-22-4), le butyl méthoxydibenzoylméthane (CAS 70356-09-1), etc.

Les absorbants UV seront utilisés seuls ou sous la forme de combinaison dont le pourcentage total dans la formule peut aller de 0,1 à 1 % en poids sec par rapport au poids total de la composition.

Parmi les additifs utilisables selon l'invention, on citera également les modificateurs de surface comme les cyclométhicones ou cyclopentasiloxanes, les diméthicones et le triméthylsiloxysilicate.

Ces additifs de surface sont généralement utilisés dans de faibles quantités dans la formulation, allant de 0,1 à 5 % en poids sec par rapport au poids total de la composition.

On citera également les additifs dits « traitants » de l'ongle utilisables dans la composition selon l'invention. Les additifs traitants peuvent représenter de 0,01% à 5% en poids sec, de préférence de 0,05% à 2% en poids sec par rapport au poids total de la composition. Parmi ces additifs « traitants », on citera :
- le formaldéhyde utilisé comme durcisseur de l'ongle,
- la vitamine E acétate (tocophéryl acétate),
- la vitamine A palmitate (rétinyl palmitate),
- les dérivés organiques ou inorganiques de calcium tels que le chlorure de calcium, le pantothénate de calcium,
- le diméthyloxobenzodioxasilane,
- la myrrhe,
- les acides aminés soufrés comme la cystéine, ses sels et leurs dérivés, la cystine, le glutathion. Les acides aminés soufrés sont en effet capables de créer des ponts de réticulation entre la kératine et les groupes SH libres de ses dérivés soufrés,
- la kératine hydrolysée d'origine végétale,
- les alpha-hydroxyacides comme l'acide citrique, l'acide ascorbique,
- la biotine,
- l'urée et la diméthylurée.

La présence de tous ces additifs permet de moduler à la fois l'aspect esthétique de la composition et ses propriétés physicochimiques.

La présente invention concerne également un vernis à ongles comportant ladite composition de l'invention telle que décrite précédemment.

Ainsi ledit vernis de l'invention répond à la réglementation CE N°1223/2009. Il est sans danger pour les consommatrices ni pour l'environnement, il présentera un taux de nitrosamines toujours inférieur à une valeur présentant un risque pour le consommateur.

En outre, la présence de la substance thixotrope inhibitrice de la nitrosamine a pour avantage de ne pas détériorer les propriétés de base du vernis à savoir sa couvrance, sa tenue, sa fluidité, sa brillance, etc.... Au contraire, ladite substance, en inhibant la nitrosamine, augmente la durée de vie, le temps de conservation, l'indice thixotropique et la fluidité du vernis.

La présente invention concerne également un procédé de préparation de ladite composition anhydre cosmétiquement acceptable pour vernis à ongles comprenant au moins une étape dans laquelle on mélange au moins un solvant organique, au moins un agent filmogène, au moins un plastifiant, au moins un agent thixotrope avec au moins une substance thixotrope inhibitrice de la nitrosamine telle que susmentionnée.

### Résultats d'expériences

Les résultats d'expérience ci-dessous permettent de prouver l'effet de l'ajout de la substance thixotrope inhibitrice de la nitrosamine dans une composition de vernis à ongle dit VAO.

### Expérience 1 :

Le tableau 1 ci-dessous montre la quantité de nitrosamine présente dans une composition de vernis à ongles dit « VAO » mesurée après 6 mois de vieillissement, avec ou sans ajout de substance inhibitrice de la nitrosamine.

Les nitrosamines détectées dans le vernis sont de type 2,2-(nitrosoimino)bisethanol (NDELA), diméthylnitrosamine (NDMA), N,N-diethyl-N-nitrosamine (NDEA) ou encore N-nitrosomorpholine (NMOR).

Les substances inhibitrices testées sont du furanéol, du maltol ou du carbonate de propylène (substance thixotrope inhibitrice de la nitrosamine exclue de la presente invention telle que revendiquée). Le furanéol et le maltol représentent chacune 0,1% en poids par rapport à la masse totale de ladite composition en VAO classique sans inhibiteur, alors que le carbonate de propylène représente 6 % en poids.

**Tableau 1 :**

| VAO ayant vieillit 6 mois | NDELA | NDMA | NDEA | NMOR | IT (indice thixotropie) |
|---|---|---|---|---|---|
| VAO sans agent | 0 | 161 | 0 | 120 | 1,59 |
| VAO + 0.1% Furaneol | 0 | 145 | 0 | 92 | 1,82 |
| VAO + 0.1% Maltol | 0 | 129 | 0 | 102 | 1,89 |
| VAO + 6% carbonate de propylène | 0 | 148 | 0 | 98 | 2,49 |

Les résultats montrent la quantité de nitrosamine mesurée après 6 mois de vieillissement en µg de nitrosamine / litre de vernis, ainsi que l'indice thixotropique.

Une diminution de NDMA et de NMOR est visible en présence de chacune des substances inhibitrices testées.

Le Maltol est la substance la plus efficace pour diminuer la formation des NDMA, tandis que le Furanéol est la plus efficace pour limiter la formation des NMOR.

En outre, en plus de posséder un fort pouvoir d'inhibition sur les NMOR, le carbonate de propylène a pour effet d'augmenter l'indice thixotropique du VAO donc de le rendre plus stable.

Ainsi, le tableau 1 permet de montrer l'effet thixotrope d'inhibition de la nitrosamine de composant de type Furanéol, Maltol et carbonate de propylène au sein d'un VAO.

La présence de ces substances au sein d'un VAO de formule classique a donc pour effet d'augmenter l'indice thixotropique du VAO et de diminuer les risques toxicologiques liés à la présence de nitrosamines, ceci sans influencer les autres caractéristiques et propriétés du vernis.

### Expérience 2 :

Le tableau 2 ci-dessous montre la quantité de nitrosamine mesurée en µg / L de VAO, d'un échantillon test, après 0, 7, 15, 18 ou 31 semaines, et, dans lequel on a ajouté à 15 semaines 0,1% de Maltol en poids par rapport au poids total de l'échantillon test de VAO.

**Tableau 2 :**

| Valeurs absolues (ppb=ug/kg=ug/L, densité du VAO~1) | | | | | |
|---|---|---|---|---|---|
| Temps (semaine) | 0 | 7 | 15 | 18 | 31 |
| NDELA | | | 0 | 0 | 0 |
| NDMA | 77 | 116 | 161 | 129 | 84 |
| NDEA | | | 0 | 0 | 4 |
| NMOR | 63 | 95 | 102 | 102 | 77 |

On constate que l'ajout de 0,1 de Maltol permet de diminuer de manière significative la production de NDMA et de NMOR au sein du VAO au cours du temps.

Plus spécifiquement pour cette expérience 2, le tableau 3 montre le pourcentage % de diminution de la quantité de NDMA et NMOR après ajout du Maltol à 15 semaines.

**Tableau 3**

| | % de diminution | | | | |
|---|---|---|---|---|---|
| Temps (semaines) | 0 | 7 | 15 | 18 | 31 |
| NDELA | | | | | |
| NDMA | 48% | 72% | 100% | 80% | 52% |
| NDEA | | | | | |
| NMOR | 62% | 93% | 100% | 100% | 75% |

Selon le tableau 3, le Maltol a bien un effet inhibiteur de la fabrication de la nitrosamine et un effet destructeur de la nitrosamine existante dans le VAO.

En effet, après 18 ou 31 semaines, c'est-à-dire après ajout de Maltol, la proportion de NDMA n'augmente pas mais au contraire diminue, ainsi, le Maltol empêche la formation de NDMA et détruit le NDMA qui était initialement présent à 15 semaines.

De la même manière, après 18 semaines, on constate que la quantité de NMOR est inchangée en présence du Maltol. En d'autres termes, il est possible que le maltol détruise autant de NMOR qu'il ne s'en fabrique entre 15 et 18 semaines d'où une stabilisation à 100%. Après 31 semaines, le Maltol présent détruit plus rapidement le NMOR que le NMOR ne se forme.

En conséquence, que ce soit envers l'une ou l'autre des catégories de nitrosamines, l'ajout de Maltol dans un VAO a pour effet d'inhiber à la fois la réaction de nitrosation et les nitrosamines.

Des résultats similaires à l'expérience 2 ont été démontrés en utilisant le carbonate de propylène ou le furanéol en remplacement du Maltol.

Il est a noté que l'effet obtenu a été augmenté en présence de la combinaison de ces trois molécules susmentionnées.

Ces résultats prouvent l'effet inhibiteur et thixotrope de ladite substance thixotrope inhibitrice de la nitrosamine présente dans la composition de l'invention.

## Revendications

1. Composition anhydre cosmétiquement acceptable pour vernis à ongles comprenant au moins un solvant organique, au moins un agent filmogène, au moins un plastifiant, au moins un agent thixotrope, **caractérisée en ce qu'**elle comporte en outre au moins une substance thixotrope inhibitrice de la nitrosamine qui consiste en une ou plusieurs molécule(s), choisie(s) parmi les molécule(s) appartenant à au moins l'une des familles suivantes :
- la famille des hydroxypyran-4-ones de formule générique (I) : 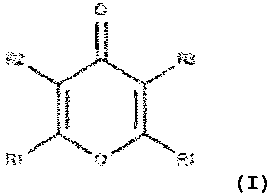 dans laquelle :
- au moins un des radicaux R1, R2, R3 ou R4 consiste en un groupement -OH, ou en un groupement -OR' avec R' étant un éther ou un ester,
- le ou les autres radicaux restants consistant en un groupement -OH, -OR', -H, un alkyl, une amine, un groupement aromatique ou un groupement aliphatique avec ou sans hétéroatome ;
- la famille des hydroxyfuran-3-one, de formule générique (II) : 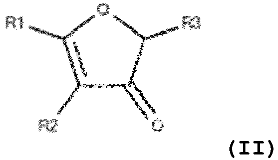
dans laquelle :
- au moins un des groupements R1, R2, ou R3 consiste en un groupement -OH, ou en un groupement -OR' étant un éther ou un ester,
- le ou les autres groupements restants consistant en un groupement -OH, -OR', -H, un alkyl, une amine, un groupement aromatique ou un groupement aliphatique avec ou sans hétéroatome.

2. Composition anhydre cosmétiquement acceptable pour vernis à ongles, selon l'une quelconque des revendications précédentes, **caractérisée en ce que** ladite substance thixotrope inhibitrice de la nitrosamine consiste en du maltol et/ou du furaneol.

3. Composition anhydre cosmétiquement acceptable pour vernis à ongles, selon l'une quelconque des revendications précédentes, **caractérisée en ce que** ladite substance thixotrope inhibitrice de la nitrosamine représente entre 0,00001% et 25% en poids par rapport au poids total de ladite composition anhydre cosmétiquement acceptable pour vernis à ongles.

4. Composition anhydre cosmétiquement acceptable pour vernis à ongles, selon l'une quelconque des revendications précédentes, **caractérisée en ce que** ledit au moins un solvant organique est choisi parmi le toluène, le xylène, l'acétate d'éthyle, l'acétate de butyle, l'acétate d'isobutyle, l'acétate de propyle, l'acétate de méthyle, une cétone, un ester, l'isopropanol, le butanol le diacétone alcool, l'éthanol dénaturé avec la méthyléthylcétone ou le diéthyléther, les hydrocarbures linéaires ou cycliques, de préférence le cyclohexane, l'heptane ou n'importe quelle combinaison de ceux-ci, de préférence le solvant organique est l'acétate d'éthyle, l'acétate de butyle ou une combinaison de ceux-ci.

5. Composition anhydre cosmétiquement acceptable pour vernis à ongles, selon l'une quelconque des revendications précédentes, **caractérisée en ce que** ledit au moins un agent filmogène est choisi parmi la cellulose, un dérivé de cellulose, une résine vinylique, une résine polyester, une résine epoxy-tosylamide, une résine acrylique, un styrène acrylique, une résine de copolymère ou n'importe quelle combinaison de ceux-ci, de préférence l'agent filmogène est la nitrocellulose.

6. Composition anhydre cosmétiquement acceptable pour vernis à ongles, selon l'une quelconque des revendications précédentes, **caractérisée en ce que** ledit au moins un plastifiant est choisi parmi l'acétyl tributyl citrate, l'acétyl triéthyl citrate, le tributyl citrate, le triéthylcitrate, le dibutyl phtalate, le triphénylphosphate, la triacétine, le triméthyl pentanyl diisobutyrate, la triéthyIhexanoïne, le sucrose benzoate, le dibutyl adipate, le diéthyl phtalate, le diisobutyl adipate, le diisopropyl adipate, le dipropylène glycol dibenzoate, le N-éthyl toluène sulfonamide, ses isomères ortho et para, le N-(2-hydroxypropyl) benzène sulfonamide et le N-(n-butyl) benzène sulfonamide ou n'importe quelle combinaison de ceux-ci.

7. Composition anhydre cosmétiquement acceptable pour vernis à ongles, selon l'une quelconque des revendications précédentes, **caractérisée en ce que** ledit au moins un agent thixotrope choisi parmi les silices pyrogénées et/ou silices hydrophiles et/ou hydrophobes et/ou argiles de type bentone, hectorite, kaolin, avec ou sans organo-modifiant ou n'importe quelle combinaison de ceux-ci.

8. Composition anhydre cosmétiquement acceptable pour vernis à ongles, selon l'une quelconque des revendications précédentes, **caractérisée en ce qu'**elle comprend en outre un ou plusieurs additifs choisis parmi les agents rhéologiques, les absorbeurs UV, les modificateurs de surface et les agents dits « traitants » et les agents de coloration.

9. Composition anhydre cosmétiquement acceptable pour vernis à ongles, selon l'une quelconque des revendications précédentes, **caractérisée en ce que** ledit au moins un solvant organique représente entre 10% et 90% en poids par rapport au poids total de ladite composition anhydre cosmétiquement acceptable pour vernis à ongles.

10. Composition anhydre cosmétiquement acceptable pour vernis à ongles, selon l'une quelconque des revendications précédentes, **caractérisée en ce que** ledit au moins un agent filmogène représente entre 5 et 40% en poids par rapport au poids total de ladite composition anhydre cosmétiquement acceptable pour vernis à ongles.

11. Composition anhydre cosmétiquement acceptable pour vernis à ongles, selon l'une quelconque des revendications précédentes, **caractérisée en ce que** ledit plastifiant représente entre 1 et 20% en poids par rapport au poids total de ladite composition anhydre cosmétiquement acceptable pour vernis à ongles.

12. Composition anhydre cosmétiquement acceptable pour vernis à ongles, selon l'une quelconque des revendications précédentes, **caractérisée en ce que** ledit au moins un agent thixotrope représente entre 0,05 et 10% en poids par rapport au poids total de ladite composition anhydre cosmétiquement acceptable pour vernis à ongles.

13. Vernis à ongles **caractérisé en ce qu'**il comprend une composition anhydre selon l'une quelconque des revendications précédentes.

14. Procédé de préparation d'une composition anhydre cosmétiquement acceptable pour vernis à ongles selon l'une des revendications 1 à 9, **caractérisé en ce qu'**il comprend au moins une étape dans laquelle on mélange au moins un solvant organique, au moins un agent filmogène, au moins un plastifiant, au moins un agent thixotrope avec au moins une substance thixotrope inhibitrice de la nitrosamine.

## Patentansprüche

1. Kosmetisch verträgliche wasserfreie Zusammensetzung für Nagellack, die wenigstens ein organisches Lösungsmittel, wenigstens einen Filmbildner, wenigstens einen Weichmacher, wenigstens ein Thixotropiermittel umfasst, **dadurch gekennzeichnet, dass** sie weiterhin wenigstens eine Nitrosamin hemmende, thixotrope Substanz aufweist, die aus einem oder mehreren Molekülen besteht, die aus den Molekülen ausgewählt sind, die zu wenigstens einer der folgenden Familien gehören:
- der Familie der Hydroxypyran-4-one der allgemeinen Formel (I): 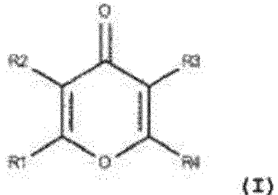 wobei:
- wenigstens einer der Reste R1, R2, R3 oder R4 aus einer -OH-Gruppe oder aus einer -OR'-Gruppe besteht, wobei R' ein Ether oder ein Ester ist,
- der oder die anderen Reste aus einer -OH-, -OR'-, -H-Gruppe, einem Alkyl, einem Amin, einer aromatischen Gruppe oder einer aliphatischen Gruppe mit oder ohne Heteroatom bestehen;
- der Familie der Hydroxyfuran-3-one der allgemeinen Formel (II): 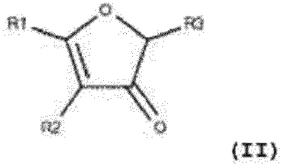 wobei:
- wenigstens eine der Gruppen R1, R2 oder R3 aus einer -OH-Gruppe oder einer - OR'-Gruppe besteht, die ein Ether oder ein Ester ist,
- wobei die andere oder die anderen Gruppen aus einer -OH-, -OR'-, -H-Gruppe, einem Alkyl, einem Amin, einer aromatischen Gruppe oder einer aliphatischen Gruppe mit oder ohne Heteroatom bestehen.

2. Kosmetisch verträgliche wasserfreie Zusammensetzung für Nagellack nach einem der vorhergehenden Ansprüche, **dadurch gekennzeichnet, dass** die Nitrosamin hemmende, thixotrope Substanz aus Maltol und/oder Furaneol besteht.

3. Kosmetisch verträgliche wasserfreie Zusammensetzung für Nagellack nach einem der vorhergehenden Ansprüche, **dadurch gekennzeichnet, dass** die Nitrosamin hemmende, thixotrope Substanz bezogen auf das Gesamtgewicht der kosmetisch verträglichen wasserfreien Zusammensetzung für Nagellack von 0,00001 % bis 25 Gew.-% ausmacht.

4. Kosmetisch verträgliche wasserfreie Zusammensetzung für Nagellack nach einem der vorhergehenden Ansprüche, **dadurch gekennzeichnet, dass** das wenigstens eine organische Lösungsmittel aus Toluol, Xylol, Ethylacetat, Butylacetat, Isobutylacetat, Propylacetat, Methylacetat, einem Keton, einem Ester, Isopropanol, Butanol, Diacetonalkohol, mit Methylethylketon oder Diethylether denaturiertem Ethanol, linearen oder cyclischen Kohlenwasserstoffen, vorzugsweise Cyclohexan, Heptan oder einer beliebigen Kombination davon ausgewählt ist, wobei das organische Lösungsmittel vorzugsweise Ethylacetat, Butylacetat oder eine Kombination davon ist.

5. Kosmetisch verträgliche wasserfreie Zusammensetzung für Nagellack nach einem der vorhergehenden Ansprüche, **dadurch gekennzeichnet, dass** der wenigstens eine Filmbildner aus Cellulose, einem Cellulosederivat, einem Vinylharz, einem Polyesterharz, einem Epoxitosylamidharz, einem Acrylharz, einem Acrylstyrol, einem Copolymerharz oder einer beliebigen Kombination davon ausgewählt ist, wobei der Filmbildner vorzugsweise Nitrocellulose ist.

6. Kosmetisch verträgliche wasserfreie Zusammensetzung für Nagellack nach einem der vorhergehenden Ansprüche, **dadurch gekennzeichnet, dass** der wenigstens eine Weichmacher aus Acetyltributylcitrat, Acetyltriethylcitrat, Tributylcitrat, Triethylcitrat, Dibutylphthalat, Triphenylphosphat, Triacetin, Trimethylpentanyldiisobutyrat, Triethylhexanoin, Saccharosebenzoat, Dibutyladipat, Diethylphthalat, Diisobutyladipat, Diisopropyladipat, Dipropylenglycoldibenzoat, N-Ethyltoluolsulfonamid, seinen ortho- und para-Isomeren, N-(2-Hydroxypropyl)benzolsulfonamid und N-(n-Butyl)benzolsulfonamid oder einer beliebigen Kombination davon ausgewählt ist.

7. Kosmetisch verträgliche wasserfreie Zusammensetzung für Nagellack nach einem der vorhergehenden Ansprüche, **dadurch gekennzeichnet, dass** das wenigstens eine Thixotropiermittel aus pyrogenen Siliciumdioxiden und/oder hydrophilen und/oder hydrophoben Siliciumdioxiden und/oder Tonen vom Typ Benton, Hectorit, Kaolin, mit oder ohne Organo-Modifiziermittel oder einer beliebigen Kombination davon ausgewählt ist.

8. Kosmetisch verträgliche wasserfreie Zusammensetzung für Nagellack nach einem der vorhergehenden Ansprüche, **dadurch gekennzeichnet, dass** sie ferner einen oder mehrere Zusatzstoffe umfasst, die aus rheologischen Mitteln, UV-Absorbern, Oberflächenmodifizierungsmitteln und Mitteln, die als "Pflegemittel" bezeichnet werden, und Farbstoffen ausgewählt sind.

9. Kosmetisch verträgliche wasserfreie Zusammensetzung für Nagellack nach einem der vorhergehenden Ansprüche, **dadurch gekennzeichnet, dass** das wenigstens eine organische Lösungsmittel bezogen auf das Gesamtgewicht der kosmetisch verträglichen wasserfreien Zusammensetzung für Nagellack von 10 % bis 90 Gew.-% ausmacht.

10. Kosmetisch verträgliche wasserfreie Zusammensetzung für Nagellack nach einem der vorhergehenden Ansprüche, **dadurch gekennzeichnet, dass** der wenigstens eine Filmbildner bezogen auf das Gesamtgewicht der kosmetisch verträglichen wasserfreien Zusammensetzung für Nagellack von 5 % bis 40 Gew.-% ausmacht.

11. Kosmetisch verträgliche wasserfreie Zusammensetzung für Nagellack nach einem der vorhergehenden Ansprüche, **dadurch gekennzeichnet, dass** der wenigstens eine Weichmacher bezogen auf das Gesamtgewicht der kosmetisch verträglichen wasserfreien Zusammensetzung für Nagellack von 1 % bis 20 Gew.-% ausmacht.

12. Kosmetisch verträgliche wasserfreie Zusammensetzung für Nagellack nach einem der vorhergehenden Ansprüche, **dadurch gekennzeichnet, dass** das wenigstens eine Thixotropiermittel bezogen auf das Gesamtgewicht der kosmetisch verträglichen wasserfreien Zusammensetzung für Nagellack von 0,05 % bis 10 Gew.-% ausmacht.

13. Nagellack, **dadurch gekennzeichnet, dass** er eine wasserfreie Zusammensetzung nach einem der vorhergehenden Ansprüche umfasst.

14. Verfahren zum Herstellen einer kosmetisch verträglichen wasserfreien Zusammensetzung für Nagellack nach einem der Ansprüche 1 bis 9, **dadurch gekennzeichnet, dass** es wenigstens einen Schritt umfasst, in dem das wenigstens eine organische Lösungsmittel, wenigstens ein Filmbildner, wenigstens ein Weichmacher, wenigstens ein Thixotropiermittel mit wenigstens einer Nitrosamin hemmenden thixotropen Substanz gemischt wird.

## Claims

1. Cosmetically acceptable anhydrous composition for nail varnish comprising at least one organic solvent, at least one film-forming agent, at least one plasticizer, at least one thixotropic agent, wherein it includes in addition at least one nitrosamine-inhibiting thixotropic substance, which consists of one or more molecule(s) chosen from the molecule(s) belonging to at least one of the following families:
the hydroxypyran-4-one family, of the generic formula (I): 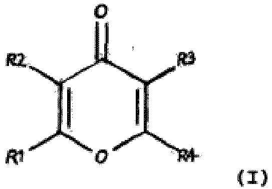
in which:
- at least one of the radicals R1, R2, R3 or R4 consists of an -OH group, or of an -OR' group, with R' being an ether or an ester,
- the other remaining radical or radicals consisting of an -OH, -OR', -H group, an alkyl, an amine, an aromatic group or an aliphatic group with or without a heteroatom;
- the hydroxyfuran-3-one family, of the generic formula (II) : 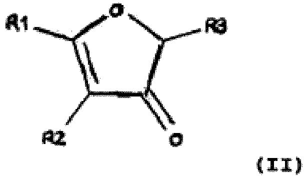
in which:
- at least one of the R1, R2 or R3 groups consists of an - OH group, or of an -OR' group, which is an ether or an ester,
- the other remaining group or groups consisting of an - OH, -OR', -H group, an alkyl, an amine, an aromatic group or an aliphatic group with or without a heteroatom.

2. Cosmetically acceptable anhydrous composition for nail varnish according to any one of the preceding claims, wherein said nitrosamine-inhibiting thixotropic substance consists of maltol and/or furaneol.

3. Cosmetically acceptable anhydrous composition for nail varnish according to any one of the preceding claims, wherein said nitrosamine-inhibiting thixotropic substance represents between 0.00001% and 25% by weight in relation to the total weight of said cosmetically acceptable anhydrous composition for nail varnish.

4. Cosmetically acceptable anhydrous composition for nail varnish according to any one of the preceding claims, wherein said at least one organic solvent is chosen from toluene, xylene, ethyl acetate, butyl acetate, isobutyl acetate, propyl acetate, methyl acetate, a ketone, an ester, isopropanol, butanol, diacetone alcohol, ethanol denatured with methyl ethyl ketone or diethyl ether, linear or cyclic hydrocarbons, preferably cyclohexane, heptane or any combination thereof, preferably the organic solvent is ethyl acetate, butyl acetate or a combination thereof.

5. Cosmetically acceptable anhydrous composition for nail varnish according to any one of the preceding claims, wherein said at least one film-forming agent is chosen from cellulose, a cellulose derivative, a vinyl resin, a polyester resin, an epoxy-tosylamide resin, an acrylic resin, an acrylic styrene, a copolymer resin or any combination thereof, preferably the film former is nitrocellulose.

6. Cosmetically acceptable anhydrous composition for nail varnish according to any one of the preceding claims, wherein said at least one plasticizer is chosen from acetyl tributyl citrate, acetyl triethyl citrate, tributyl citrate, triethyl citrate, dibutyl phthalate, triphenylphosphate, triacetin, trimethyl pentanyl diisobutyrate, triethylhexanoin, sucrose benzoate, dibutyl adipate, diethyl phthalate, diisobutyl adipate, diisopropyl adipate, dipropylene glycol dibenzoate, N-ethyl toluene sulfonamide, its ortho- and para-isomers, N-(2-hydroxypropyl) benzene sulfonamide and N-(n-butyl) benzene sulfonamide or any combination thereof.

7. Cosmetically acceptable anhydrous composition for nail varnish according to any one of the preceding claims, wherein said at least one thixotropic agent is chosen from the pyrogenic silicas and/or the hydrophilic and/or hydrophobic silicas and/or clays such as bentone, hectorite, kaolin, with or without organo-modifier, or any combination thereof.

8. Cosmetically acceptable anhydrous composition for nail varnish according to any one of the preceding claims, wherein it comprises in addition one or more additives chosen from the rheological agents, the UV absorbers, the surface modifiers and the so-called "treating" agents and the coloring agents.

9. Cosmetically acceptable anhydrous composition for nail varnish according to any one of the preceding claims, wherein said at least one organic solvent represents between 10% and 90% by weight in relation to the total weight of said cosmetically acceptable anhydrous composition for nail varnish.

10. Cosmetically acceptable anhydrous composition for nail varnish according to any one of the preceding claims, wherein said at least one film-forming agent represents between 5 and 40% by weight in relation to the total weight of said cosmetically acceptable anhydrous composition for nail varnish.

11. Cosmetically acceptable anhydrous composition for nail varnish according to any one of the preceding claims, wherein said plasticizer represents between 1 and 20% by weight in relation to the total weight of said cosmetically acceptable anhydrous composition for nail varnish.

12. Cosmetically acceptable anhydrous composition for nail varnish according to any one of the preceding claims, wherein said at least one thixotropic agent represents between 0.05 and 10% by weight in relation to the total weight of said cosmetically acceptable anhydrous composition for nail varnish.

13. Nail varnish, wherein it comprises an anhydrous composition according to any one of the preceding claims.

14. Method for preparing a cosmetically acceptable anhydrous composition for nail varnish according to one of claims 1 to 9, wherein it comprises at least one step in which at least one organic solvent, at least one film-forming agent, at least one plasticizer, at least one thixotropic agent are mixed with at least one nitrosamine-inhibiting thixotropic substance.
